# EUROPEAN PATENT SPECIFICATION

(11) **EP 3 275 559 B1**
(45) Date of publication and mention of the grant of the patent: **15.09.2021**
(21) Application number: 16305971.0
(22) Date of filing: 27.07.2016
(51) Int. Cl.: B05B 15/00

(54) **COATING SYSTEM WITH AN ULTRASONIC SPRAY HEAD AND ELECTROSTATIC FIELD**
BESCHICHTUNGSSYSTEM MIT EINEM ULTRASCHALLZERSTÄUBER UND ELEKTROSTATISCHEM FELD
SYSTÈME DE REVÊTEMENT AVEC UN ATOMISEUR À ULTRASONS ET UN CHAMP ÉLECTROSTATIQUE

(43) Date of publication of application: 31.01.2018
(73) Proprietor: EXEL INDUSTRIES, 51200 Epernay (FR)
(72) Inventor: COLRAT, Michel, 75009 PARIS (FR); PAGOT, Rémi, 75009 PARIS (FR); ENGLE, Robb, 75009 PARIS (FR)
(74) Representative: Lavoix

(56) References cited:
- EP-A1- 1 110 617
- EP-A2- 0 253 539
- WO-A1-2010/097539
- FR-A1- 2 657 794
- JP-A- H 091 004
- JP-A- H06 106 096
- JP-A- 2004 148 239
- US-A- 4 929 319
- US-A- 5 026 463
- US-A1- 2009 181 159
- US-A1- 2013 034 723
- US-A1- 2013 284 833

## Description

### TECHNICAL FIELD OF THE INVENTION

This invention relates to a coating system for coating a workpiece with a liquid coating product.

### BACKGROUND OF THE INVENTION

Ultrasonic liquid atomization has been used so far for spraying a liquid coating product onto a workpiece to be coated. In "Ultrasonic Liquid Atomization Theory and Application" (Partridge Hill Publishers - 1998 and 2006) Harvey L. Berger considers the application of electrostatics to ultrasonic liquid atomization and explains that implementing electrostatics onto ultrasonic atomization would theoretically bring a very high efficiency to the control of the spray application, even with voltage much lower than those used in conventional electrostatic spraying.

Since then, some applications of ultrasonic atomization with electrostatics have been considered. For instance, in a paper "Deposition of CulnS2 films by electrostatic field assisted ultrasonic spray pyrolysis" (Solar Energy Materials and Solar Cells 95 (2011) 245-249), Dong - Yeup Li and JunHo Kim discloses a case where a liquid to be sprayed is excited by an ultrasonic probe in order to be converted into an aerosol which is conveyed by a carrying gas towards an outlet of a tube, in order to be used for spray pyrolysis deposition. Such a system does not allow precisely controlling the flow rate nor the density of the aerosol and, in its path between the bath where it is formed and the outlet of the tube, the aerosol might agglomerate, so that the size of the droplets used for coating an object cannot be accurately controlled because of coalescence generally observed between droplets. The coating might be rough or non homogeneous and some defects may be generated on the coated surface.

In "Deposition of Ni-CGO composite anodes by electrostatic assisted ultrasonic spray pyrolysis method" (Materials Research Bulletin 42 (2007) 1674-1682), Jing-Chiang Chen et al. consider creating an aerosol and conveying it in a glass tube toward an nozzle facing a copper plate which supports a substrate to be coated. Here again, there is a risk of agglomeration of the droplets of the aerosol.

These academic works would not be easy to use in an industrial environment because the coating deposition is highly dependent on many factors, such as the geometry and length of a tube conveying an aerosol.

On the other hand, in a known system, the electrostatic field is generally created between an electrode and the object to be coated, this electrostatic field having a field line distribution which is not optimized for the workpiece to be coated. Under such circumstances, a substantial part of the atomized product might end up outside of the surface to be coated, which is detrimental in terms of pollution and from an economic stand point.

It is also known from US-A-5 026 463 to use a piezo-electric ultrasonic vibrator system and some Corona electrodes to treat a sheet material. The electrostatic field distribution is not precisely controlled.

### SUMMARY OF THE INVENTION

The invention aims at solving these problems with a new coating system which makes use of the versatility of an ultrasonic head and which allows optimizing the distribution of electrostatic field lines, thus optimizing the coating efficiency.

To this end, the invention concerns a coating system for coating a workpiece with a liquid coating product, this coating system including:
- a grounded ultrasonic spray head for generating droplets of coating product,
- an electrode for generating an electrostatic field between the electrode and the ultrasonic spray head and
- a high voltage generator connected to the electrode for supplying the electrode with high voltage.

The high voltage generator is integrated in a sub-module which includes also a discharge resistor and connection means, said sub-module defining two housings, where the high voltage generator and the discharge resistor are respectively received, and a connection housing where a connector is received to connect the high voltage generator and the discharge resistor. Moreover, the electrode is shaped according to the workpiece geometry and supported by a base part which includes the high-voltage generator.

Thanks to the invention, the electrode allows precisely controlling the electrostatic field line distribution towards the workpiece, thus the path of the coating product droplets towards the workpiece. More precisely, the electrode acts as a conformator for an electric field which guides the droplets towards the workpiece to be coated

According to further aspects of the invention which are advantageous but not compulsory, the coating system might incorporate one or several features of the dependent claims, taken in any technically admissible combination. In particular, thanks to the substitutable electrode, the shape of the electrode can be customized according to the actual geometry of the workpiece to be coated, which allows building the electrostatic field lines closely around the workpiece, thus avoiding, or limiting to a great extent, an overspray phenomenon.

### BRIEF DESCRIPTION OF THE DRAWINGS

The invention will be better understood on the basis of the following description which is given in correspondence with the annexed figures and as an illustrative example, without restricting the object of the invention. In the annexed figure:
- figure 1 is a perspective view of a coating system according to the invention;
- figure 2 is a perspective view, on a larger scale, of some components of the system of figure 1, in a first configuration of use;
- figure 3 is a perspective view similar to figure 2 when the system is in a second configuration of use;
- figure 4 is a schematic representation of the wiring used for controlling an electrode module of the system of figures 1 to 3;
- figure 5 is a perspective view of the electrode module in a first configuration of use corresponding to the configuration of the system on figure 2;
- figure 6 is a cut-away view of the electrode module in the configuration of figure 5;
- figure 7 is an enlarged partial cut view along line VII-VII on figure 6;
- figure 8 is an exploded view of the electrode module;
- figure 9 is a cut-away view similar to figure 6 when the electrode module is in a second configuration of use; and
- figure 10 is a cut away view similar to figure 6 when the electrode module is in a third configuration of use corresponding to the configuration of the system on figure 3.

### DETAILED DESCRIPTION OF SOME EMBODIMENTS

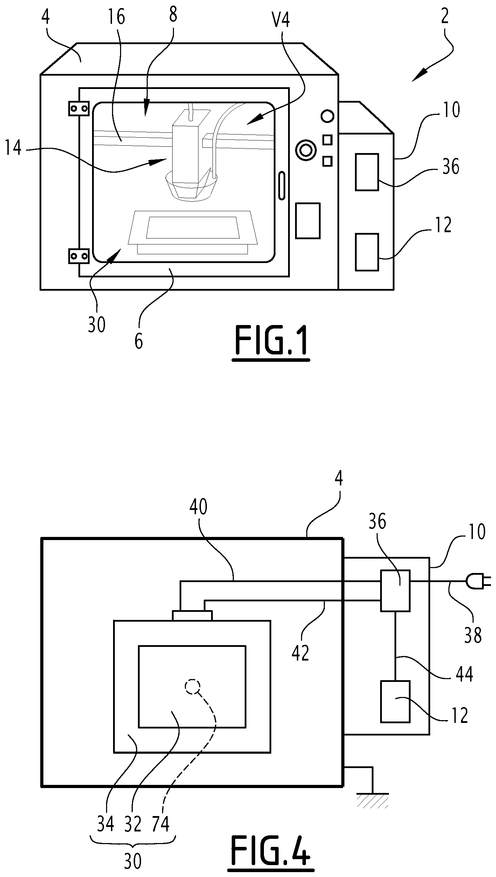

The coating system 2 represented in figures 1 to 7 includes an enclosure 4 which defines an internal volume V4 closed by a door 6 provided with a transparent window 8 made of mineral or organic glass, which allows checking volume V4 through door 6.

Alternatively, door 6 is not transparent.

Enclosure 4 forms a cabin for a coating process to be implemented with coating system 2.

Coating system 2 also includes a control cabinet 10 which comprises, amongst others, a programmable logic controller or PLC 12. On figure 1, the front panel of control cabinet 10 has been removed in order to show PLC 12.

Coating system 2 includes an ultrasonic spray head 14 which is supported by a 3 axis Cartesian robot and which has its longitudinal axis X14 oriented vertically. Ultrasonic head is directed downwardly towards a workpiece W1 to be coated which lies underneath ultrasonic head 14.

In another embodiment, the spray head can be handled by a six axis robot allowing that axis X14 presents an angle with the vertical for an optimal position of the spray head in front of the workpiece surface to be coated, especially for volumic/3D workpieces.

The ultrasonic head frequency can be chosen in the whole ultrasonic frequency range from 20kHz to 10 MHz, and for instance at the following frequencies : 25 kHz, 35 kHz, 48 kHz, 60 kHz, 120 kHz, 180 kHz, 250 kHz. The size of the droplets generated by ultrasonic spray heads, expressed in Number Median Diameter (NMD or DN0.5) are typically given as follows: 69µm (25khz), 50µm (35kHZ), 38µm (48kHZ), 32µm (60kHZ), 18µm (120kHZ), 12µm (180kHZ), 8µm (250kHZ).

Ultrasonic spray head 14 may be of any commercially available type.

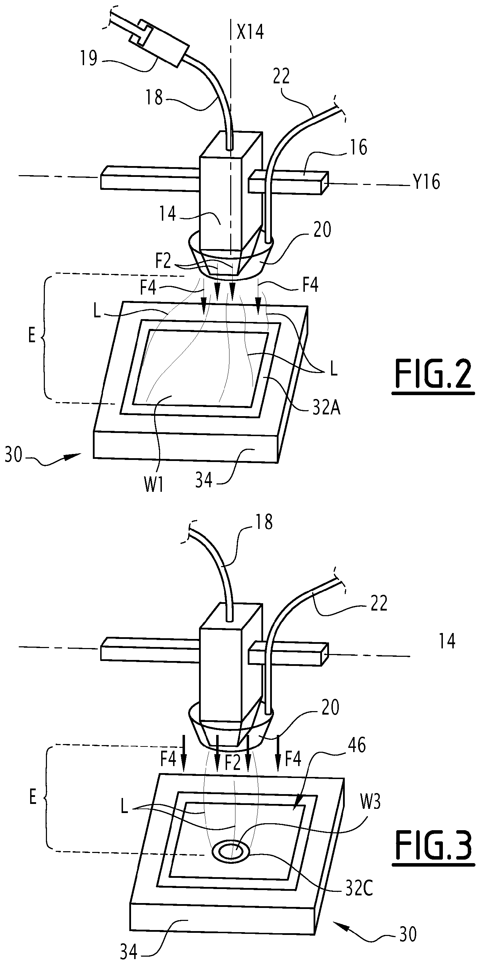

A pipe 18 feeds ultrasonic head 14 with a liquid to be atomized and a non-represented vibrating member integrated within head 14 is actuated to atomize this coating product when workpiece W1 is actually located under head 14. On figure 2, arrows F2 represent the flow of droplets of atomized coating product coming out of ultrasonic head 14 and directed towards workpiece W1.

Pipe 18 is fed with coating product from a metering pump 19 visible on figure 2 only and represented as a syringe. Preferably, metering pump 19 has a movable piston. Alternatively, other types of metering devices can be used to feed ultrasonic head 14 via pipe 18, such as a gear pump. Using a metering device to feed ultrasonic head 14 allows a precise control of the amount of coating product, thus a precise control of flow F2 of droplets.

In order to better control the shape of the spray of droplets exiting head 14, a shaping gas unit 20 is mounted around the lower extremity of ultrasonic head 14. This unit 20 is fed with air via a pipe 22 and expels a flow of air, represented by arrows F4 on figure 2, around the flow of droplets F2. This avoids that this flow diverges radially with respect to axis X14. Alternatively, a gas different from air can be used in shaping unit 20.

Head 14 is movable in three directions of the space with a 3 axis Cartesian robot represented by a guide rail 16.

In order to enhance the effectiveness of the coating of workpiece W1, an electrostatic field E is generated between spray head 14 and an electrode.

To this end, an electrode module 30 is located within volume V4, together with ultrasonic head 14 and the 3 axis cartesian robot. The electrode module 30 supports workpiece W1. In other words, workpiece W1 lies on electrode module 30. Workpiece W1 is electrostatically charged by contact with electrode 32A.

This electrode module 30 has a flat upper surface constituted by an electrode 32A made of a sheet of electrically conductive material, in particular a metal, such as a ferrous metal (steel, stainless steel...) or a non-ferrous metal (aluminum, copper...) and their alloys.

This electrode 32A lies on a base part 34 of electrode module 30, which is made of an electrically insulating material, such as a synthetic material, for instance polypropylene (PP), polyoxymethylene (POM), polytetrafluoroethylene (PTFE), polyvinylidene difluoride (PVDF), polyvinyl chloride (PVC). Base part 34 insulates workpiece W1 and electrode 32A from the ground potential, preventing any electrical leak from the electrode.

As shown on figure 4, electrode module 30 is controlled by a high voltage controller 36 located in control cabinet 10. More precisely, high voltage controller 36 is connected to a power grid via a first cable 38 and to electrode module 30 via a second cable 40. A third cable 42 connects electrode module 32 to enclosure 4, which is grounded.

On the other hand, a control cable 44 allows PLC 12 to control high voltage controller 36 according to a pre-established control sequence and in a way consistent with applicable safety rules.

Ultrasonic spray head 14 is grounded via a non represented grounding cable and a non represented ultrasonic connector, which can be considered as "double grounding". Actually, this is important insofar as the vibrating member of ultrasonic head 14 is an electrical device whose operation could be disturbed if it were submitted to a high voltage. Thus, the general layout of coating system 2, where high voltage is applied at the level of electrode module 30, not at the level of ultrasonic head 14, is advantageous.

When electrode module 30 is supplied with an electrical current via cable 40, electrostatic field E is generated between ultrasonic head 14 and electrode 32. Electrostatic field lines L extend between ultrasonic head 14, on the one hand, and electrode 32 and workpiece W1, on the other hand.

Electrode 32A is charged, with a high voltage in the range between 0.1 kV and 100 kV, and preferably between 5 kV and 30 kV. Assuming the high voltage is negative, the droplets of atomized coating product exiting ultrasonic spray head 14 are charged positively by influence, so that they follow the field lines L towards electrode 32, thus towards workpiece W1 which lies on top of electrode 32A.

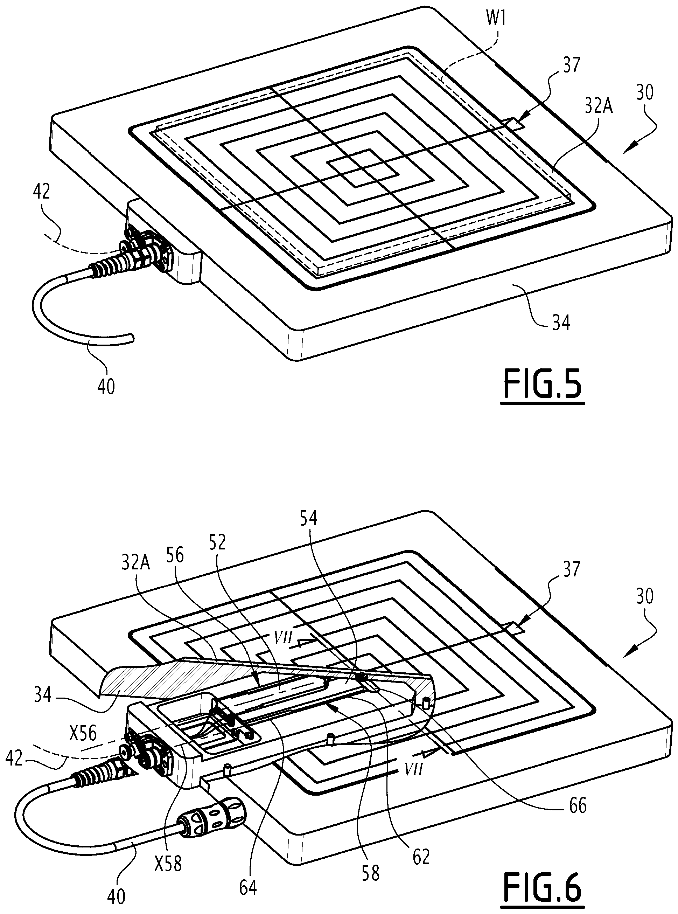

In the example of figure 2, workpiece W1 is a flat piece, for instance a solar cell glass cover. On figure 5, workpiece W1 is represented in chain-dotted lines in order to fully show electrode 32A.

In such a case, the shape of electrode 32A is flat and rectangular, with an edge having substantially the same shape as the edge of workpiece W1, as shown on figure 5.

For instance, electrode 32A can be a rectangle with the same proportions as the rectangle defined by workpiece W1 and with a surface area S32 comprised between 80% and 5000%, preferably between 100% and 125%, of the surface area SW of the workpiece W1.

More precisely, surface area S32 can be smaller than or equal to surface area SW, with the ration S32/SW in the range between 0,8 and 1. Alternatively, surface areas S32 and SW can be equal or substantially equal, with ratio S32/SW in the range between 0,95 and 1,05. According to another approach, surface area S32 may be larger than or equal to surface area SW, with ratio S32/SW in the range between 1 and 50.

Under such circumstances, the portion of electrode 32A which is "visible" from spray head 14 around workpiece W1 can be reduced like in the configuration of figure 2, so that all the field lines L end up and close around workpiece W1. Thus, droplets coming out of ultrasonic head 14 are efficiently directed by electrostatic field E towards workpiece W1.

As can be derived from figures 5 to 10, base part 34 defines a top cavity 46 which forms an area with a length L46 and a width W46 corresponding to the maximum dimensions of an electrode 32A and any other electrode to be used in electrode module 30.

In the configuration of figures 2, 5 6 and 8, electrode 32A has the maximum possible dimensions, so that it fills cavity 46 up. A notch 47 allows accessing laterally cavity 46 to exert a lifting effort on electrode 32A when it must be separated from base part 34, e.g. with a tool such as the tip of a screwdriver or even the finger tip.

Base part 34 also defines a recess 48 which accommodates a sub-module 50 adapted to convert the current received from high voltage controller 36 via cable 40 into a high voltage to be applied to electrode 32.

Sub-module 50 includes a high voltage generator 52 made by a cascade of diodes, according to a known technique in the field of electrostatic spraying, in particular in hand guns. Sub-module 50 includes an insulative body 54 which defines a first elongated housing 56 extending along a first axis X56. Body 54 also defines a second elongated housing 58 which extends along a second axis X58 parallel to axis X56. Actually, axes X56 and X58 can be non-parallel with any orientation. In a preferred configuration, axes X56 and X58 are substantially parallel, that is converge or diverge with an angle between them of less than 30°, so that they open out on the same side of body 54. This configuration makes a very neat connection since the supply cable 40 may bring together the high voltage power and the grounded reference. A connection housing 60 connects housings 56 and 58 perpendicularly to axes X56 and X58. Thus, housings 56, 58 and 60 together define a U shaped volume for accommodating the high voltage supply means of sub-module 50.

A discharge resistor 62 is located within housing 58, together with a connecting rod 64. On the other hand, a connector 66 is accommodated within housing 60.

A spring 68 is interposed between high voltage generator 52 and connector 66. Another spring 70 is interposed between connector 66 and resistor 62. Thus, connector 66 connects high voltage generator 52 and discharge resistor 62 with the help of springs 68 and 70.

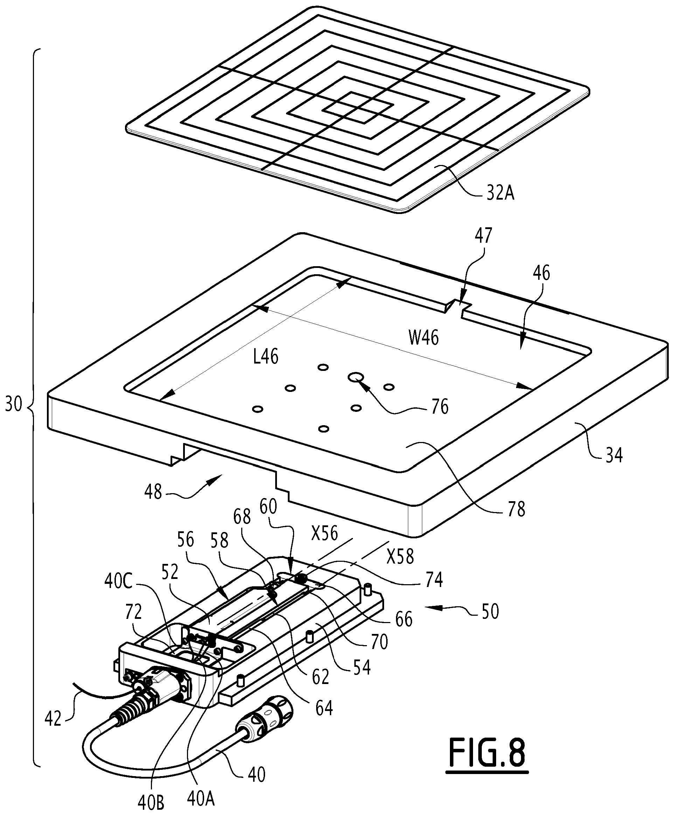

A ground plate 72 is located at one end of body 54 where housings 56 and 58 open out, opposite from housing 60. This allows connecting high voltage generator 52 and connecting rod 64 to the ground via ground cable 42, whereas high voltage generator 52 is also connected to cable 40 via individual conductors 40A, 40B and 40C. For the sake of clarity, connectors 40A, 40B and 40C are represented only on figure 8.

Connector 66 bears a contact member 74 which goes through an opening 76 of a wall 78 of base part 34 separating cavity 46 from recess 48. Thus, when sub-module 50 is mounted within recess 48, contact member 74 is flush with wall 78 or protrudes into cavity or area 46, so that it can apply to any electrode installed within cavity or area 46 a high voltage originating from high voltage generator 52.

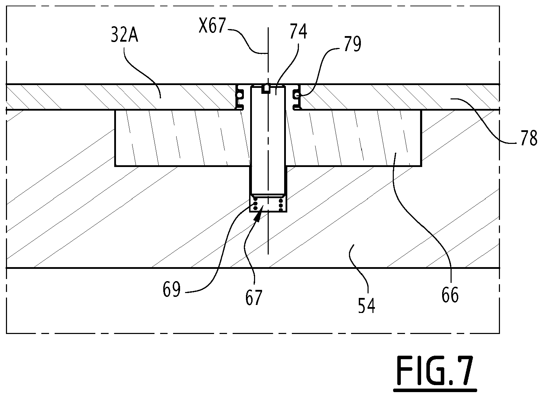

As shown on figure 7, contact member 74 is formed by an electrically conductive stud housed in a vertical bore 67 of connector 66. Connector 66 is immobilized within wall 78 by an O-ring 79. The vertical position of stud 74 within bore 67 can be adjusted along the central axis X67 of bore 67. A spring 69 is advantageously provided at the closed end of bore 67, which biases stud against electrode 32A.

As can be derived from figures 5, 6, 9 and 10, electrodes with different geometries can be used with base part 34 and sub-module 50.

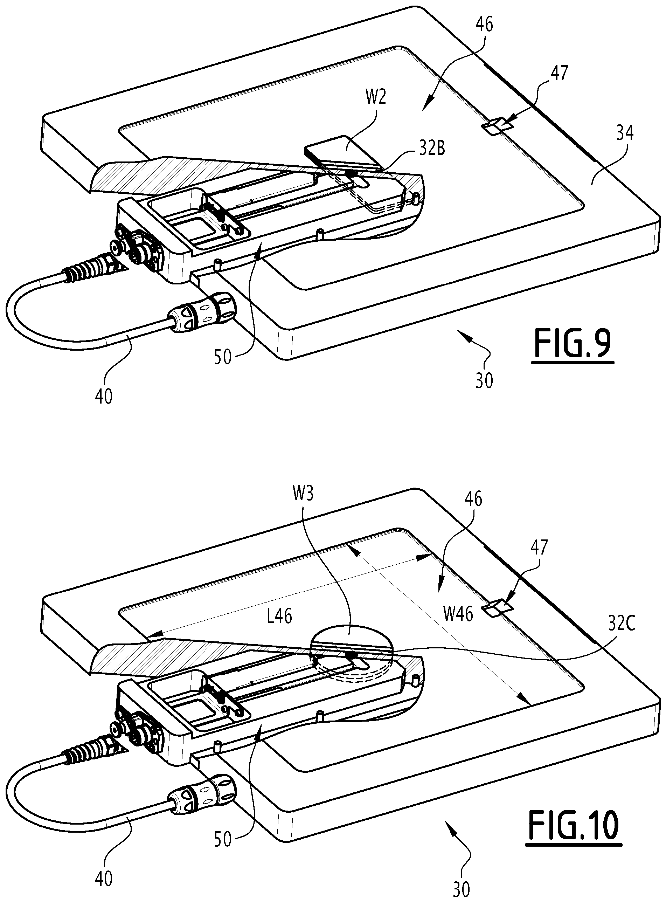

For example, the rectangular electrode 32A used in the configuration of figure 2 for a large workpiece W1 of a rectangular shape can be replaced with an electrode 32B having a flat and rectangular shape adapted to a workpiece W2 with smaller dimensions, such as a screen of a cellphone, as shown on figure 9. Provided that electrode 32B is positioned in the center part of cavity 46, so that it contacts member 74, this electrode is also suitable for creating an electrostatic field with ultrasonic head 14, so that the operation described with respect to figure 2 can be obtained an electrostatic field lines close on electrode 32B around workpiece W2.

An electrode 32C can also be used in case a workpiece W3, in the form of a disc or a portion of a sphere, is used, as shown on figures 3 and 10. For example, the workpiece W3 can be an optical lens to be coated with different coating layers such as a hydrophobic layer, a top coat protective layer, a hard coating or even anti-reflective coating layers. If workpiece W3 is a disc, electrode 32C is flat. If workpiece W3 is in the form of a portion of a sphere, electrode 32C is preferably curved or warped to conform to the shape of workpiece W3. In this second case, electrode 32C may protrude upwardly with respect to cavity 46, which still forms an area for accommodating electrode 32C.

On figures 9 and 10, for easier understanding, the global shape of workpieces W2 and W3 and the global shape of electrodes 32B and 32C are shown partly in dotted lines.

As shown on figure 3, the electrostatic field E established between electrode 32C and ultrasonic head 14, which is grounded, gives rise to field lines L which extend between items 14 and 32C, so that the droplets leaving ultrasonic head 14 in the form of flow F2 are guided by these field lines towards workpiece W3.

In this example, the ratio of the respective diameters of workpiece W3 and electrode 32C can be chosen between 0.8 and 2.

Other shapes can be selected for the electrode placed on area 46, depending on the actual geometry of the workpiece to be treated. For instance, a triangular electrode or a polygonal electrode, with more than four sides, can be manufactured in order to follow the contour of the workpiece to be treated which can be polygonal or rounded, in particular with an oval shape. Also, a tridimensional or curved shape design of the electrode can be chosen. For instance, a hemispherical or paraboloid electrode can be designed in order to support a hemispherical or paraboloid optical lens. By extension, any volumic regular or irregular shape electrode can be chosen depending on the geometry of the workpiece to be treated.

Actually, the edge of the electrode 32A, 32B, 32C or equivalent is advantageously an image of the contour of the workpiece, as seen from ultrasonic head 14. In other words, the edge of the electrode can be defined so as to evenly distribute the droplets around the contour of the workpiece, and increase the transfer efficiency of the product to the workpiece.

Advantageously, the edge of the electrode 32A, 32B or 32C is an exact image of the contour of the workpiece, where "exact image" means that the difference between an area of a surface bordered by the edge of the electrode and an area bordered by the contour of the workpiece is less than 10% of the area bordered by the contour.

On the other hand, the geometry of the electrode 32A, 32B or 32C can be adapted by increasing the exposed surface of the electrode in this region, the exposed surface of the electrode being the surface which is visible around the workpiece from ultrasonic head 14, so as to precisely control the coating profile on the edge of the workpiece.

According to a non represented alternative embodiment, the electrode may be formed by the workpiece itself. For instance, in the first embodiment, workpiece W1 to W3 may be in direct electrical contact with contact member 74 or with brush 82.

The features of the embodiments and variants considered here-above can be combined in order to generate new embodiments of the invention.

The scope of the invention is defined by the appended claims. In particular, an air ejection unit similar to unit 20 can be used in the second to fourth embodiments.

## Claims

1. A coating system (2) for coating a workpiece (W1-W5, W5') with a liquid coating product, the coating system including a grounded ultrasonic head (14) for generating droplets of coating product, wherein the coating system further includes:
- an electrode (32A-32C) for generating an electrostatic field (E) between the electrode and the ultrasonic spray head, and
- a high-voltage generator (52) connected to the electrode for supplying the electrode with high voltage,
**characterized in that**
- the high voltage generator (52) is integrated in a sub-module (50) which includes also a discharge resistor (62) and connection means (66, 68, 70), said sub-module defining two housings (56, 58), where the high voltage generator and the discharge resistor are respectively received, and a connection housing (60) where a connector (66) is received to connect the high voltage generator and the discharge resistor and
- the electrode (32A-32C) is shaped according to the workpiece geometry and supported by a base part (34) which includes the high-voltage generator (52).

2. The coating system according to claim 1, wherein the electrode (32A-32C) is substitutable on the basis of the geometry of the workpiece (W1-W5, W5').

3. The coating system according to one of the preceding claims, wherein an edge of the electrode (32A-32C) is an image of the contour of the workpiece (W1-W3) as seen from the ultrasonic head (14), preferably an exact image of this contour.

4. The coating system according to any preceding claim, wherein the electrode (32A-32F) supports the workpiece (W1-W4) and is in contact with the workpiece.

5. The coating system according to any preceding claim, wherein the base part (34) insulates the workpiece (W1-W3) from the ground potential, preventing any electrical leak from the electrode (32A-32C).

6. The coating system according to any preceding claim, wherein the base part (34) defines an area (46) configured for accommodating electrodes (32A-32C) of different shapes.

7. The coating system of claim 6, wherein an electrical contact member (74) is located in the area (46) for connecting the high voltage generator (52) to an electrode (32A-32C) accommodated in the area.

8. The coating system according to claim 7, wherein the electrical contact member (74) is movable along an axis (X67) non parallel to an electrode (32A-32C) accommodated in the area (46) and biased towards this electrode.

9. The coating system according to any one of claims 5 to 7, wherein the area configured for accommodating electrodes is a cavity (46) defined on top of the base part (34) .

10. The coating system according to claim 1, wherein the electrode is formed by the workpiece.

11. The coating system according to any preceding claims, wherein the ultrasonic head (14) is provided with an air ejection unit (20) configured to deliver a jet (F4) of shaping air around the droplets (F2) leaving the ultrasonic head.

12. The coating system according to any preceding claim, wherein it includes a metering device (19) for feeding the ultrasonic head (14) with liquid product in controlled amount, in particular in the form of a pump with a movable piston.

13. The coating system of claim 7, wherein the electrical contact member (74) is supported by, and electrically connected to, the connector (66) received in the connection housing (60).

14. The coating system of claim 13, wherein the electrical contact member is formed by an electrically conductive stud (74) housed in a vertical bore (67) of the connector (66).

15. The coating systems of claims 8 and 14, wherein a spring (69) is provided at a close end of the bore (67) and biaises the stud (67) against the electrode (32A) .

## Patentansprüche

1. Beschichtungssystem (2) zum Beschichten eines Werkstücks (W1-W5, W5') mit einem flüssigen Beschichtungsprodukt, wobei das Beschichtungssystem einen geerdeten Ultraschallkopf (14) zum Erzeugen von Tröpfchen des Beschichtungsprodukts beinhaltet, wobei das Beschichtungssystem ferner Folgendes beinhaltet:
- eine Elektrode (32A-32C) zum Erzeugen eines elektrostatischen Felds (E) zwischen der Elektrode und dem Ultraschallsprühkopf, und
- einen Hochspannungsgenerator (52), der mit der Elektrode verbunden ist, um die Elektrode mit Hochspannung zu versorgen,
**dadurch gekennzeichnet, dass**
- der Hochspannungsgenerator (52) in ein Untermodul (50) integriert ist, das auch einen Entladewiderstand (62) und Verbindungseinrichtungen (66, 68, 70) beinhaltet, wobei das Untermodul zwei Gehäuse (56, 58), in denen jeweils der Hochspannungsgenerator und der Entladewiderstand aufgenommen sind, und ein Verbindungsgehäuse (60), in dem ein Verbinder (66) aufgenommen ist, um den Hochspannungsgenerator und den Entladewiderstand zu verbinden, definiert, und
- die Elektrode (32A-32C) entsprechend der Werkstückgeometrie geformt ist und von einem Basisteil (34) getragen wird, das den Hochspannungsgenerator (52) beinhaltet.

2. Beschichtungssystem nach Anspruch 1, wobei die Elektrode (32A-32C) basierend auf der Geometrie des Werkstücks (W1-W5, W5') austauschbar ist.

3. Beschichtungssystem nach einem vorherigen Anspruch, wobei eine Kante der Elektrode (32A-32C) ein Bild der Kontur des Werkstücks (W1-W3), von dem Ultraschallkopf (14) aus gesehen, ist, vorzugsweise ein exaktes Bild dieser Kontur.

4. Beschichtungssystem nach einem vorherigen Anspruch, wobei die Elektrode (32A-32F) das Werkstück (W1-W4) trägt und in Kontakt mit dem Werkstück ist.

5. Beschichtungssystem nach einem vorherigen Anspruch, wobei das Basisteil (34) das Werkstück (W1-W3) von dem Erdungspotential isoliert, wodurch einen elektrischen Fehlerstrom von der Elektrode (32A-32C) verhindert wird.

6. Beschichtungssystem nach einem vorherigen Anspruch, wobei das Basisteil (34) einen Bereich (46) definiert, der konfiguriert ist, um Elektroden (32A-32C) unterschiedlicher Formen unterzubringen.

7. Beschichtungssystem nach Anspruch 6, wobei sich in dem Bereich (46) ein elektrisches Kontaktelement (74) zum Verbinden des Hochspannungsgenerators (52) mit einer Elektrode (32A-32C), die in dem Bereich untergebracht ist, befindet.

8. Beschichtungssystem nach Anspruch 7, wobei das elektrische Kontaktelement (74) entlang einer Achse (X67) beweglich ist, die nicht parallel zu einer Elektrode (32A-32C) ist, die in dem Bereich (46) untergebracht ist und gegen diese Elektrode vorgespannt ist.

9. Beschichtungssystem nach einem der Ansprüche 5 bis 7, wobei der Bereich, der zum Unterbringen von Elektroden konfiguriert ist, ein Hohlraum (46) ist, der auf der Oberseite des Basisteils (34) definiert ist.

10. Beschichtungssystem nach Anspruch 1, wobei die Elektrode durch das Werkstück gebildet ist.

11. Beschichtungssystem nach einem vorherigen Anspruch, wobei der Ultraschallkopf (14) mit einer Luftausstoßeinheit (20) versehen ist, die konfiguriert ist, um einen Strahl (F4) formgebender Luft um die Tröpfchen (F2), die den Ultraschallkopf verlassen, zuzuführen.

12. Beschichtungssystem nach einem vorherigen Anspruch, **dadurch gekennzeichnet, dass** es eine Dosiereinrichtung (19) zum Zuführen flüssigen Produkts in kontrollierter Menge zu dem Ultraschallkopf (14), insbesondere in Form einer Pumpe mit beweglichem Kolben, beinhaltet.

13. Beschichtungssystem nach Anspruch 7, wobei das elektrische Kontaktelement (74) von dem Verbinder (66), der in dem Verbindungsgehäuse (60) aufgenommen ist, getragen wird und damit elektrisch verbunden ist.

14. Beschichtungssystem nach Anspruch 13, wobei das elektrische Kontaktelement durch einen elektrisch leitenden Stift (74) gebildet ist, der in einer vertikalen Bohrung (67) des Verbinders (66) untergebracht ist.

15. Beschichtungssysteme nach Ansprüche 8 und 14, wobei an einem nahen Ende der Bohrung (67) eine Feder (69) bereitgestellt ist und den Bolzen (67) gegen die Elektrode (32A) vorspannt.

## Revendications

1. Un système de revêtement (2) destiné à revêtir une pièce (W1-W5, W5') d'un produit de revêtement liquide, le système de revêtement comprenant une tête à ultrasons mise à la terre (14) destinée à générer des gouttelettes de produit de revêtement, dans lequel le système de revêtement comprend en outre :
- une électrode (32A-32C) destinée à générer un champ électrostatique (E) entre l'électrode et la tête de pulvérisation à ultrasons, et
- un générateur de haute tension (52) connecté à l'électrode et destiné à alimenter l'électrode en haute tension,
**caractérisé en ce que**
- le générateur de haute tension (52) est intégré dans un sous-module (50) qui comprend également une résistance de décharge (62) et des moyens de connexion (66, 68, 70), ledit sous-module définissant deux boîtiers (56, 58), où le générateur de haute tension et la résistance de décharge sont respectivement logés, et un boîtier de connexion (60) où un connecteur (66) est logé pour connecter le générateur de haute tension et la résistance de décharge et
- l'électrode (32A-32C) est formée selon la géométrie de la pièce et supportée par un socle (34) qui comprend le générateur de haute tension (52).

2. Le système de revêtement selon la revendication 1, dans lequel l'électrode (32A-32C) est substituable en fonction de la géométrie de la pièce (W1-W5, W5').

3. Le système de revêtement selon l'une des revendications précédentes, dans lequel un bord de l'électrode (32A-32C) est une image du contour de la pièce (W1-W3) comme vu depuis la tête à ultrasons (14), de préférence une image exacte de ce contour.

4. Le système de revêtement selon l'une quelconque des revendications précédentes, dans lequel l'électrode (32A-32F) supporte la pièce (W1-W4) et est en contact avec la pièce.

5. Le système de revêtement selon l'une quelconque des revendications précédentes, dans lequel le socle (34) isole la pièce (W1-W3) du potentiel de terre, empêchant toute fuite électrique depuis l'électrode (32A-32C).

6. Le système de revêtement selon l'une quelconque des revendications précédentes, dans lequel le socle (34) définit une zone (46) configurée pour accueillir des électrodes (32A-32C) de différentes formes.

7. Le système de revêtement de la revendication 6, dans lequel un élément de contact électrique (74) est situé dans la zone (46) afin de connecter le générateur de haute tension (52) à une électrode (32A-32C) logée dans la zone.

8. Le système de revêtement selon la revendication 7, dans lequel l'élément de contact électrique (74) est mobile le long d'un axe (X67) non parallèle à une électrode (32A-32C) logée dans la zone (46) et sollicité vers cette électrode.

9. Le système de revêtement selon l'une quelconque des revendications 5 à 7, dans lequel la zone configurée pour accueillir des électrodes est une cavité (46) définie sur le dessus du socle (34).

10. Le système de revêtement selon la revendication 1, dans lequel l'électrode est formée par la pièce.

11. Le système de revêtement selon l'une quelconque des revendications précédentes, dans lequel la tête à ultrasons (14) est pourvue d'une unité d'éjection d'air (20) conçue pour émettre un jet (F4) d'air de mise en forme autour des gouttelettes (F2) quittant la tête à ultrasons.

12. Le système de revêtement selon l'une quelconque des revendications précédentes, dans lequel est inclus un dispositif de dosage (19) destiné à alimenter la tête à ultrasons (14) avec un produit liquide en quantité contrôlée, en particulier sous la forme d'une pompe dotée d'un piston mobile.

13. Le système de revêtement de la revendication 7, dans lequel l'élément de contact électrique (74) est supporté par, et connecté électriquement au, connecteur (66) logé dans le boîtier de connexion (60).

14. Le système de revêtement de la revendication 13, dans lequel l'élément de contact électrique est formé par une tige conductrice d'électricité (74) logée dans un alésage vertical (67) du connecteur (66).

15. Les systèmes de revêtement des revendications 8 et 14, dans lesquels un ressort (69) est disposé au niveau d'une extrémité fermée de l'alésage (67) et sollicite la tige (67) contre l'électrode (32A).
